(19) Europäisches Patentamt
European Patent Office
Office européen des brevets

(11) Publication number : **0 487 237 A1**

(12) **EUROPEAN PATENT APPLICATION**

(21) Application number : **91310439.4**

(22) Date of filing : **12.11.91**

(51) Int. Cl.⁵ : **C07C 213/10,** C07C 217/32, C07B 57/00

(30) Priority : **17.11.90 JP 312492/90**

(43) Date of publication of application : **27.05.92 Bulletin 92/22**

(84) Designated Contracting States : **CH DE GB LI**

(71) Applicant : **Nitto Denko Co. Ltd.**
**1-2, 1-chome Shimohozumi**
**Ibaraki City, Osaka 567 (JP)**

(72) Inventor : **Nakazono, Yutaka**
**Nitto Denko Co. Ltd., 1-2 Simohozumi 1-chome**
**Inaraki City, Osaka 567 (JP)**
Inventor : **Omata, Tetsuo**
**Nitto Denko Co. Ltd., 1-2 Simohozumi 1-chome**
**Inaraki City, Osaka 567 (JP)**
Inventor : **Senda, Shuji**
**Nitto Denko Co. Ltd., 1-2 Simohozumi 1-chome**
**Inaraki City, Osaka 567 (JP)**

(74) Representative : **Copp, David Christopher et al**
**Dummett Copp & Co. 14 The Square**
**Martlesham Heath**
**Ipswich Suffolk IP5 7SL (GB)**

(54) **New optically active metoprolol tartrate salts, and method of preparing them, and method of preparing optically activemetoprolol from the tartrate salts.**

(57) This invention relates to new optically active metoprolol tartrate salts, (2S)-1-isopropylamino-3-[p-(2-methoxyethyl)phenoxy]-2-propanol-(2S′, 3S′)-O,O-di-p-toluoyltartrate salts (formula 1), and (2R)-1-isopropylamino-3-[p-(2-methoxyethyl)phenoxy]-2-propanol-(2R′, 3R′)-O,O-di-p-toluoyltartrate salts (formula 2), and preparation thereof and of optically active metoprolol from the tartrate salts which is caracterized in that optically active substances of metoprolol, which function as cures for essential hypertension, angina pectoris, and tachycardiac arhythmia can be provided readily and industrially.

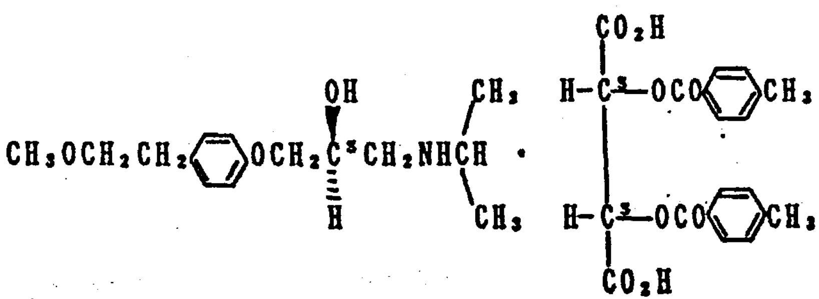

(formula 1)

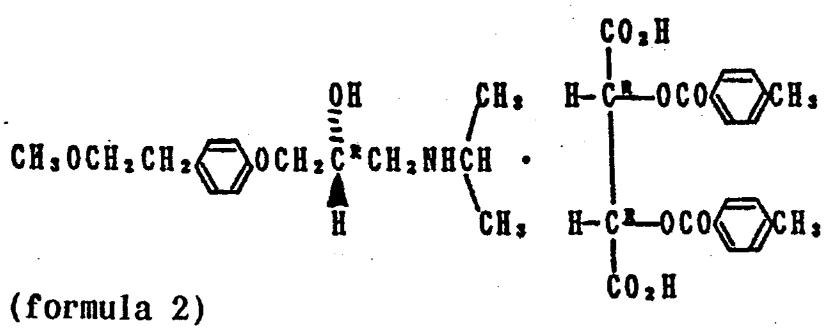

(formula 2)

EP 0 487 237 A1

(Background of the Invention)

It is known that ß-receptor blockades such as metoprolol (formula 3) bring excellent cures for angina pectoris, hypertension, angiopathic neurasthenia, cardiopathy with some types of arhythmia, and angiopathy.

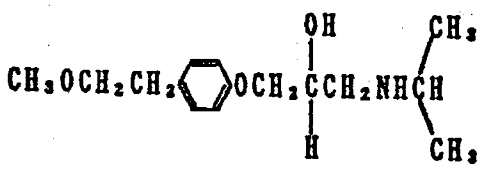

(formula 3)

The pharmacologically active form of such a ß-receptor blockade is generally either of the optical antipodes. Therefore, function of one optical antipode can be determined by synthesizing the pure optical antipode.

(Industrial Field of the Invention)

The present invention relates to new optically active metoprolol tartrate salts and preparation thereof and of optically active metoprolol from the tartrate salts. The objective of the invention is to provide (2S)-1-isopropylamino-3-[p-(2-methoxyethyl)phenoxy]-2-propanol-(2S′, 3S′)-O,O-di-p-toluoyltartrate salts and (2R)-1-isopropylamino-3-[p-(2-methoxyethyl)phenoxy]-2-propanol-(2R′,3R′)-O,O-di-p-toluoyltartrate salts and preparation thereof, and thereby to provide a method for readily and industrially preparing optically active substances of metoprolol, which function as cures for essential hypertension, angina pectoris, and tachycardiac arhythmia.

(Description of Prior Art and Problems to be Solved by the Invention)

Some examples of synthesis for pure optical antipodes or similar compounds are disclosed in British Patent No, 1,598,867 and 1,598,668, and Tokukaisho-61-47447.

Such syntheses are, however, rather complicated and require a lot of steps, thus having low production efficiency. Namely, they are not applicable to the industry.

Another known method is to obtain optical antipodes by optically separating the racemic modification (J. Chromatog. 316 605 -616 (1984): and J.Chromatog, 350 1, 328-331 (1985)).

The latter method separates the racemic modification by gas chromatography or liquid chromatography, and is thus not suitable for mass production or industrial production.

(Brief Explanation of the Drawings)

Fig.1 is an infrared absorption spectral atlas of (2S)-1-isopropylamino-3-[p-(2-methoxyethyl)phenoxy]-2-propanol-(2S′, 3S′)-O,O-di-p-toluoyltartrate salts.

Fig.2 is a proton nuclear magnetic resonance spectral atlas of (2S)-1-isopropylamino-3-[p-(2-methoxyethyl)phenoxy]-2-propanol -(2S′, 3S′)-O,O-di-p-toluoyltartrate salts.

Fig.3 is an infrared absorption spectral atlas of (+)-metoprolol.

Fig.4 is a proton nuclear magnetic resonance spectral atlas of (+)- metoprolol.

(Detailed Description of the Invention)

Hereinafter, this inventional embodiments are discribed in detail.

Optically active metoprolol tartrate salts which ralate to this invention are (2S)-1-isopropylamino-3-[P-(2-methoxyethyl)phenoxy]-2-propanol-(2S′, 3S′)-O,O-di-p-toluoyltartrate salts (formula 1) and (2R)-1-isopropylamino-3-[P-(2-methoxyethyl)phenoxy]-2-propanol-(2R′, 3R′)-O,O-di-p-toluoyltartrate salts.

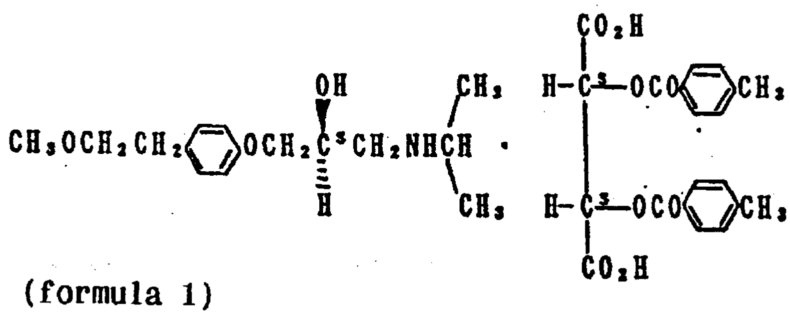

(formula 1)

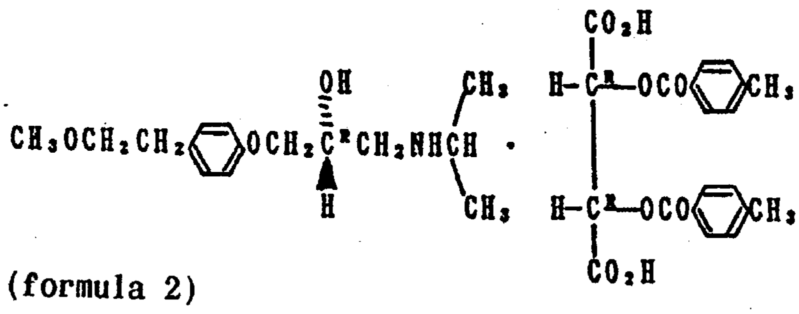

(formula 2)

(Here, S and R denote the configuration.)

The substance of (2S)-1-isopropylamino-3-[P-(2-methoxyethyl)phenoxy]-2-propanol-(2S′, 3S′)-O,O-di-p-toluoyltartrate salts (formula 1) is white crystals having a melting point between 96.0 °C and 97.0 °C.

$[\alpha]^{25}$ was +90.6 ° (C=1.13, methanol).

The infrared absorption spectra of the salt showed the absorption at wavelength ($cm^{-1}$) of 3400(br), 3020(m), 2980(m), 2920(m), 2860(m), 1720(s), 1610(s), 1510(s), 1380(m), 1290(s), 1250(s), 1180(s), 1110(s) and 750(s) (see Fig.1).

The measurement was performed by KBr tablet method.

The δ values of the proton nuclear magnetic resonance spectra (400MHz, $CD_3$ OD) were 1.276(6H,dd,J = 2.0, 6.4 Hz), 2.381 (6H, s), 2.774(2H, t, J = 6.8Hz), 3.040(1H, dd, J = 9.5, 12.2 Hz), 3.25 through 3.40 (4H, m), 3.544(2H, t, J = 7.1Hz), 3.884 (2H, ddd, J = 5.1, 10.0, 23.0 Hz), 4.15 through 4.25(1H,m), 5.874(2H,S), 6.816(2H,d,J = 8.5Hz), 7.113(2H,d,J = 8.5Hz), 7.263 (4H,d,J = 8.3Hz), 8.003(4H, d, J = 8.3Hz) (see Fig. 2).

Moreover, the result of the elemental analysis was 92.90% C, 6.57% H and 2.11%N.

The rational formula of the resultant compound is determined to be $C_{35}H_{43}NO_{11}$ as the calculated values of $C_{35}H_{43}NO_{11}$ agrees with 92.72% C, 6.58% H and 2.14% N.

From the above-mentioned results, the salt is determined to be (2S)-1-isopropylamino-3-[P-(2-methoxyethyl)phenoxy]-2-propanol--(2S′, 3S′)-O,O-di-p-toluoyltartrate salts (formula 1).

And, the substance of (2R)-1-isopropylamino-3-[P-(2-methoxyethyl)phenoxy]-2-propanol-(2R′, 3R′)-O,O-di-p-toluoyltartrate salts (formula 2) is white crystals having a melting point between 95.5 °C and 96.5 °C.

$[\alpha]^{25}$ was -90.4 ° (C=0.467 methanol).

The infrared absorption apectral atlas and the proton nuclear magnetic resonance spectral atlas were the same as (2S)-1-isopropylamino-3-[P-(2-methoxyethyl)phenoxy]-2-propanol-(2S′, 3S′)-O,O-di-p-toluoyltartrate salts (formula 1).

The result of the element analysis was 92.70% C, 6.60% H, and 2.13% N.

The rational formula of the resultant compound is determined to be $C_{35}H_{43}NO_{11}$ as the calculated values of $C_{35}H_{43}NO_{11}$ agrees with 92. 72% C, 6. 58% H, and 2. 14% N.

From the above-mentioned results, the salt is determined to be (2R)-1-isopropylamino-3-[P-(2-methoxyethyl)phenoxy]-2-propanol-(2R′,3R′)-O,O-di-p-toluoyltartrate salts (formula 2).

Hereinafter, the methods of preparing these salt will be described.

A starting material is non-pure optically active metoprolol (formula 3), that is, recemic metoprolol containing identical quantities of optical antipodes or metoprolol containing different quantities of optical antipodes.

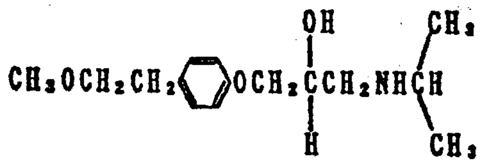

(formula 3)

The racemic metoprolol (formula 3) is prepared from 3-[p-(2-methoxyethyl)phenoxy]-1,2-propanediol as the starting material according to the method disclosed in Tokukaisho-58-159446.

The other starting material is an S-tartrate salts, that is, (+)-(2S,3S)-O,O-di-p-toluoyl tartaric acid (formula 4) or an R-tartrate salts, that is, (-)-(2R,3R)-O,O-di-ptoluoyl tartaric acid (formula 5).

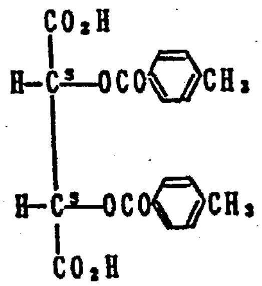

(formula 4)

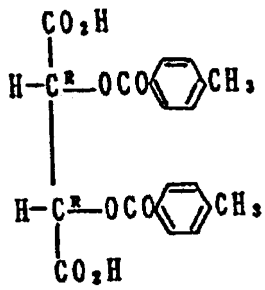

(formula 5)

(Here, S and R denote the configuration.)

Metoprolol (formula 3) reacts with the S-tartrate salts (formula 4) to yield (2S)-1-isopropylamino-3-[P-(2-methoxyethyl)phenoxy]-2-propanol-(2S′, 3S′)-O,O-di-p-toluoyltartrate salts (formula 1) and diastereomeric salts thereof.

Metoprolol (formula 3) reacts with the S-tartrate salts (formula 5) to yield (2R)-1-isopropylamino-3-[P-(2-methoxyethyl)phenoxy]-2-propanol-(2R′, 3R′)-O,O-di-p-toluoyltartrate salts (formula 2) and diastereomeric salts thereof.

Solvents favorably used in the reaction are any polar solvents. More preferable examples are: alcohol solvents such as methanol and ethanol; halogen solvents such as dichloromethane and chloroform; and ether solvents such as diethyl ether, diisopropyl ether, tetrahydrofuran, and dioxane. Dichloromethane is, however, most preferable.

One equivalent of the tartrate salts (fromula 4 and 5) is preferably mixed with metoprolol (formula 3).

The preferable temperature for the reaction ranges from 0 °C to the boiling point of the solvent, and is especially between 0°C and room temperature.

Compound of optically active metoprolol tartrate salts (fromula 1 and 2) with diastereomeric salts is recrystallized in the solvent. The diastereomeric salts are separated from the compound and optically active metoprolol tartrate salts relating to the invention (fromula 1 and 2) is obtained.

That is, (2R)-1-isopropylamino-3-[p-(2-methoxyethyl) phenoxy]-2-propanol-(2S′,3S′)-O,O-di-p-toluoyltartrate salts (formula 1) is separated from the diastereomeric salt by the recrystallization, and (2R)-1-isopropylamino-3-[p-(2-methoxyethyl) phenoxy]-2-propanol-(2R′,3R′)-O,O-di-p-toluoyltartrate salts (formula 2) is separated from the diastereomeric salt.

Solvents favorably used in the recrystallization are, for example, halogen solvents such as dichloromethane

and chloroform; and ether solvents such as diethyl ether, diisopropyl ether, tetrahydrofuran, and dioxane, aromatic solvents such as benzene and toluene, alcohol solvents such as methanol and ethanol, hydrocarbonic solvents such as hexane and pentane and mixed solvent of them.

The obtained optically active metoprolol tartrate salts (formula 1 and 2) is the optically active substance of high purity.

This optically active metoprolol tartrate salts (formula 1 and 2) is easily hydrolyzed with alkali such as sodium hydroxide and potassium hydroxide in a solvent such as mixed solvent of ether and water to be optically active metoprolol (formula 6).

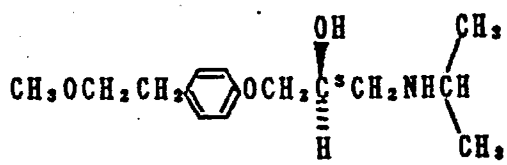

(formula 6)

(Here, S denotes the configuration.)

Optically active metoprolol tartrate salts (formula 2) is to be optically active metoprolol (formula 7) in the same manner.

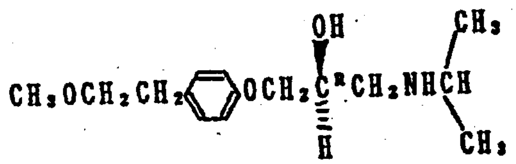

(formula 7)

(Here, R denotes the configuration.)

These obtained metoprolol (formula 6 and 7) are optically active substance of high purity.

Hereinafter, the effects of this invention will be described more clearly according to the detailed examples.

(Example 1)

Synthesis of (2S)-1-isopropylamino-3-[p-(2-methoxyethyl) phenoxy]-2-propanol-(2S′,3S′)-O,O-di-p-toluoyltartrate salts.

(±)-metoprolol (300g; 1.12 mol) was dissolved in methanol (500ml).

Monohydrate of (+)-(2S, 3S)-O,O-di-p-toluoyl tartaric acid (454g; 1.12 mol) was added to the methanol at room temperature and stirred for thirty minutes for complete dissolution.

The mixed solution was concentrated under reduced pressure to give 731g of the viscous residue.

The residue was recrystallized in a 1:1 solvent of dichloromethane and diethylether to yield 225g of (2S)-1--isopropylamino-3-[p-(2-methoxyethyl)phenoxy]-2-propanol-(2S′,3S′)-O,O-di-p-toluoyltartrate salts (yield: 31%).

The optical purity of the resultant tartrate salts was 99%e.e.

The optical purity was measured by using an HPLC with an optically active substance separation column such as CHIRALCEL OD etc. (Other examples were also measured in the same manner.)

The resultant compound was white crystals having a melting point between 96.0°C and 97.0 °C.

$[\alpha]^{25}$ was + 90.6° (C=1.13; methanol).

The infrared absorption spectra of the compound showed the absorption at wavelengths ($cm^{-1}$) of 3400(br), 3020(m), 2980(m), 2920(m), 2860(m), 1720(s), 1610(s), 1510(s), 1380(m), 1290(s), 1250(s), 1180(s), 1110(s), and 750(s) (see Fig.1).

The measurement was perfomed by KBr tablet method.

The δ values of the proton nuclear magnetic resonance spectra (400MHz, $CD_3OD$) were 1.276 (6H:dd;J = 2.0, 6.4 Hz), 2.381 (6H, S), 2.774 (2H,t,J = 6.8 Hz), 3.040 (1H,dd,J = 9.5, 12.2 Hz), 3.25 through 3.40 (4H, m) 3.544 (2H,t,J = 7.1 Hz), 3.884 (2H,ddd, J = 5.1, 10.0, 23.0 Hz), 4.15 through 4.25 (1H,m), 5.874(2H,S), 6.816

(2H,d,J = 8.5Hz), 7.113 (2H,d,J = 8.5Hz), 7.263 (4H,d,J = 8.3 Hz), 8.003 (4H,d,J = 8.3Hz) (see Fig.2).

The result of the elemental analysis was 92.90% C, 6.57% H, and 2.11% N.

Since the above result well agrees with calculated values of $C_{35}H_{43}NO_{11}$ (C:92.72% ; H:6.58% ; and N:2.14%), the rational formula of the resultant compound is determined to be $C_{35}H_{43}NO_{11}$.

(Example 2)

Synthesis of (2R)-1-isopropylamino-3-[p-(2-methoxyethyl)phenoxy]-2-propanol-(2R′,3R′)-O,O-di-p-toluoyltartrate salts.

Except using monohydrate of (-)-(2R, 3R)-O-di-p-toluoyl tartaric acid instead of monohydrate of (+)-(2S, 3S)-O-di-p-toluoyl tartaric acid in the same manner of Example 1, 262g of (2R)-1-isopropylamino-3-[p-(2-methoxyethyl)phenoxy]-2-propanol-(2R′,3R′)-O,O-di-p-toluoyltartrate salts (yield: 36%) was obtained.

The optical purity of the resultant tartrate salts was 99% e.e.

The resultant compound was white crystals having a melting point between 95.5°C and 96.5 °C.

$[\alpha]^{25}$ was -90.4 ° (C=0.467; methanol).

The infrared absorption spectral atlas and proton nuclear magnetic resonance spectral atlas were same as (2S)-1-isopropylamino-3-[p-(2-methoxyethyl)phenoxy]-2-propanol-(2S′,3S′)-O,O-di-p-toluoyltartrate salts of Example 1.

The result of the elemental analysis was 92.70% C, 6.60% H, and 2.13% N.

Since the above result well agrees with calculated values of $C_{35}H_{43}NO_{11}$ (C:92.72% ; H:6.58% ; and N:2.14%), the rational formula of the resultant compound is determined to be $C_{35}H_{43}NO_{11}$.

(Example 3)

Synthesis of (+)-metoprolol.

(2S)-1-isopropylamino-3-[p-(2-methoxyethyl)phenoxy]-2-propanol-(2S′,3S′)-O,O-di-p-toluoyltartrate salts (225g, 344mmol) obtained in Example 1 was mixed in a heterogeneous solvent of sodium hydroxide (688ml) and diethyl ether (1,200ml) and was strongly stirred at chilled temperature for one hour.

Separated the organic layer, extracted the water layer twice with diethyl ether, mixed them with the separated organic layer and dried them with sodium sulfate.

After the filtration, 82g of white crystals, (+)-metoprolol (yield: 90%), was obtained by concentrating.

The optical purity was 99% e.e.

The resultant compound had a melting point 44.5°C and $[\alpha]^{25}$ was + 0.47° (C=1.82, methanol).

The infrared absorption spectra of the compound showed the absorption at wavelength ($cm^{-1}$) of 3420(br), 2960(s), 2920(s), 2860(s), 1605(m), 1510(s), 1450(m), 1380(m), 1240(s), 1175(m), 1110(s), and 1020 (m) (see Fig.3).

The measurement was performed by KBr tablet method.

The $\delta$ values of the proton nuclear magnetic resonance spectra (400MHz, $CD_3C\ell_3$) were 1.073 (6H:d;J = 6.3 Hz), 2.40 (br,3H), 2.702 (1H,dd,J = 7.8, 12.0 Hz), 2.75 through 2.90 (3H, m), 3.333 (3H,S), 3.546 (2H,t,J = 7.1Hz), 3.90 through 4.05 (3H,m), 6.829(2H,m), 7.112 (2H,m) (see Fig.4).

The result of the elemental analysis was 67.15% C, 9.35% H, and 5.20% N.

Since the above result well agrees with calculated values of $C_{15}H_{25}NO_3$ (C:67.44% : H:9.36% and N:5.24%), the rational formula of the resultant compound is determined to be $C_{15}H_{25}NO_3$.

(Example 4)

In the same manner as Example 3, 99g of (-)-metoprolol (yield: 93%) was obtained from 262g of (2R)-1-isopropylamino-3-[p-(2-methoxyethyl)phenoxy]-2-propanol-(2R′,3R′)-O,O-di-p-toluoyltartrate salts which was obtained in Example 2.

The optical purity was 99% e.e.

The resultant compound had melting point 44.5 °C and $[\alpha]^{25}$ was - 0,47° (C=1.50; methanol).

The infrared absorption spectral atlas and the proton nuclear magnetic resonance spectral atlas of the compound were same as (+)-metoprorol which was obtained in Example 3.

The result of the elemental analysis was 67.43% C, 9.37% H, and 5.20% N.

Since the above result well agrees with calculated values of $C_{15}H_{25}NO_3$ (C:67.44% ; H:9.36% ; and N:5.24%), the rational formula of the resultant compound is determined to be $C_{15}H_{25}NO_3$.

This inventional new optically active metoprolol tartrate salts, the preparation of them, and the preparation of optically active metoprolol using the salts are the method of comprising the steps of:

reacting metoprolol containing new optically active metoprolol tartrate salts which are (2S)-1-isopropylamino- 3-[p-(2-methoxyethyl)phenoxy]-2-propanol-(2S′,3S′)-O,O-di-p-toluoyltartrate salts and (2R)-1-isopropylamino-3-[p-(2-methoxyethyl)phenoxy]-2-propanol-(2R′,3R′)-O,O-di-p-toluoyltartrate salts and optical antipodes with (+)-(2S,3S)-O,O-di-p-toluoyltartrate salts in a solvent to produce a salt; and

recrystallizing said salt in a solvent to obtain said (2S)-1-isopropylamino- 3-[p-(2-methoxyethyl)phenoxy]-2-propanol-(2S′,3S′)-O,O-di-p-toluoyltartrate salts,

and are the method of preparing optically active metoprolol tartrates salts which is caracterized by the steps of:

reacting metoprolol containing both of optical antipodes with (-)-(2R,3R)-O,O-di-p-toluoyl tartaric acid in a solvent to produce a salt; and

recrystallizing said salt in the solvent to obtain said (2R)-1-isopropylamino-3-[p-(2-methoxyethyl)phenoxy]-2-propanol-(2R′,3R′)-O,O-di-p-toluoyltartrate salts, and are the methods which is caractrized by hydrolyzing optically active metoprolol tartrates salts to obtain each optically active metoprolol, so that an easy and mass production of optically active metoprolol is available.

## Claims

**(1)** Optically active metoprolol tartrate salts, (2S)-1-isopropylamino -3-[p-(2-methoxyethyl)phenoxy]-2-propanol-(2S′, 3S′)-O-,O-di-p-toluoylatartrate salts (formula 1).

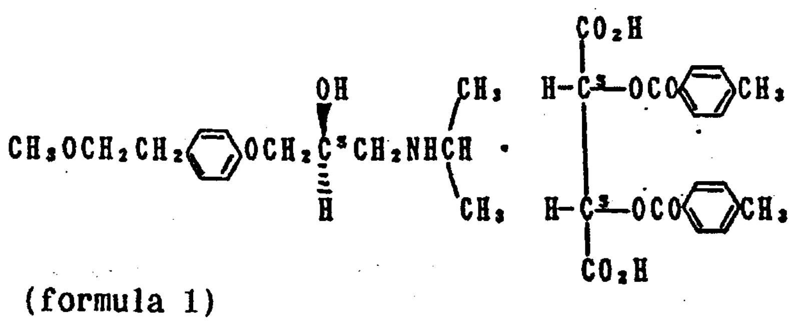

(formula 1)

**(2)** Optically active metoprolol tartrate salts, (2R)-1-isopropylamino-3-[p-(2-methoxyethyl)phenoxy]-2-propanol-(2R′,3R′) -O,O-di-p-toluoylatartrate salts (formula 2).

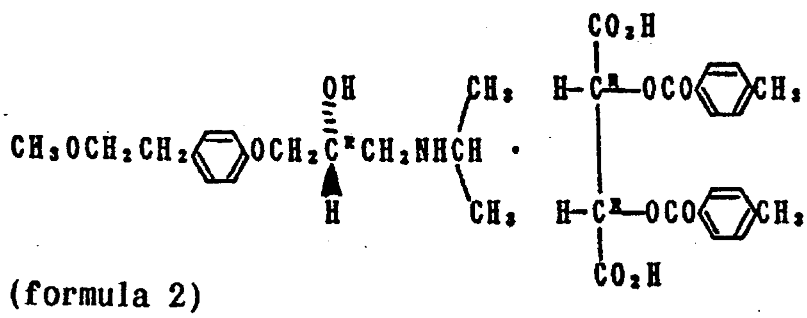

(formula 2)

**(3)** Preparation of optically active metoprolol tartrate salts (formula 1) as set forth in Claim 1, comprising the steps of:

preparing metoprolol containing both of optical antipodes (formula 3) react with (+)-(2S,3S)-O,O-di-p-toluoyl tartrate salts (formula 4) in a solvent to produce a salt; and

recrystallizing said salt in the solvent to obtain said optically active metoprolol tartrate salts.

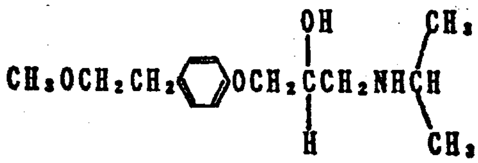

(formula 3)

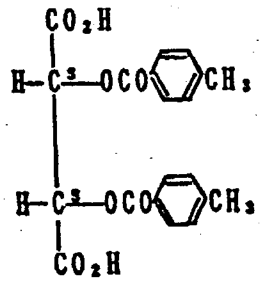

(formula 4)

(Here, S and R denote the configuration.)

**(4)** Preparation of optically active metoprolol tartrate salts (formula 2) as set forth in Claim 2, comprising the steps of:

preparing metoprolol containing both of optical antipodes (formula 3) react with (-)-(2R,3R)-O,O-di-p-toluoyl tartrate salts (formula 5) in a solvent to produce a salt; and

recrystallizing said salt in the solvent of obtain said optically active metoprolol tartrate salts.

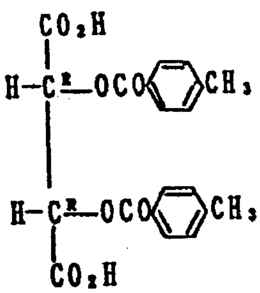

(formula 5)

(Here, S and R denote the configuration.)

**(5)** Preparation of optically active metoprolol (formula 6) by hydrolyzing optically active metoprolol tartrate salts (formula 1).

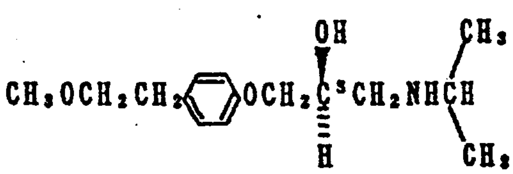

(formula 6)

**(6)** Preparation of optically active metoprolol (formula 7) by hydrolyzing optically active metoprolol tartrate salts (formula 2).

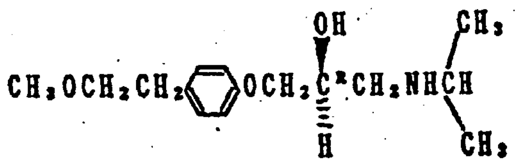

(formula 7)

(Here, S and R denote the configuration.)

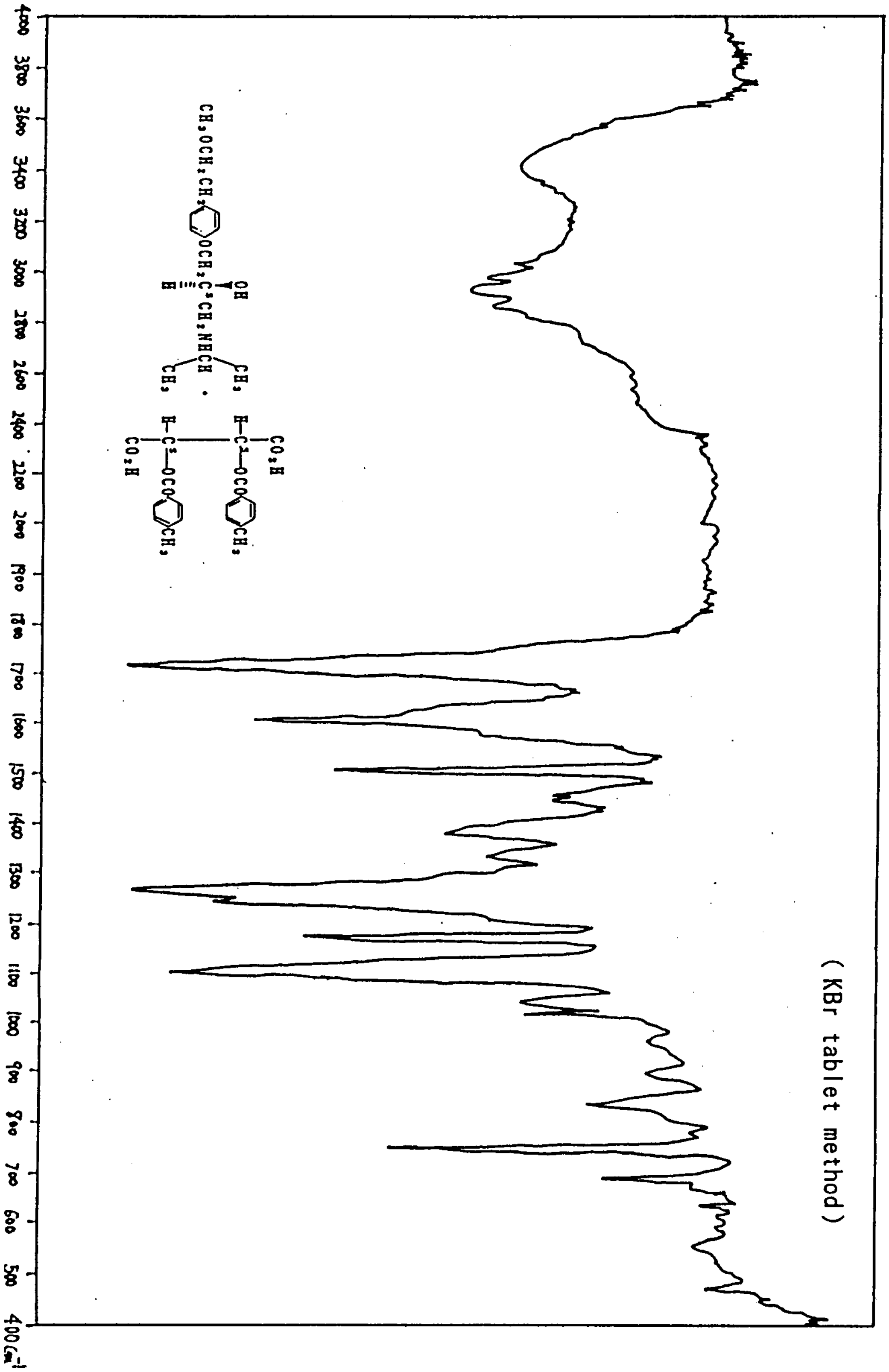
(KBr tablet method)
4000 3800 3600 3400 3200 3000 2800 2600 2400 2200 2000 1900 1800 1700 1600 1500 1400 1300 1200 1100 1000 900 800 700 600 500 400 cm⁻¹

Fig. 1

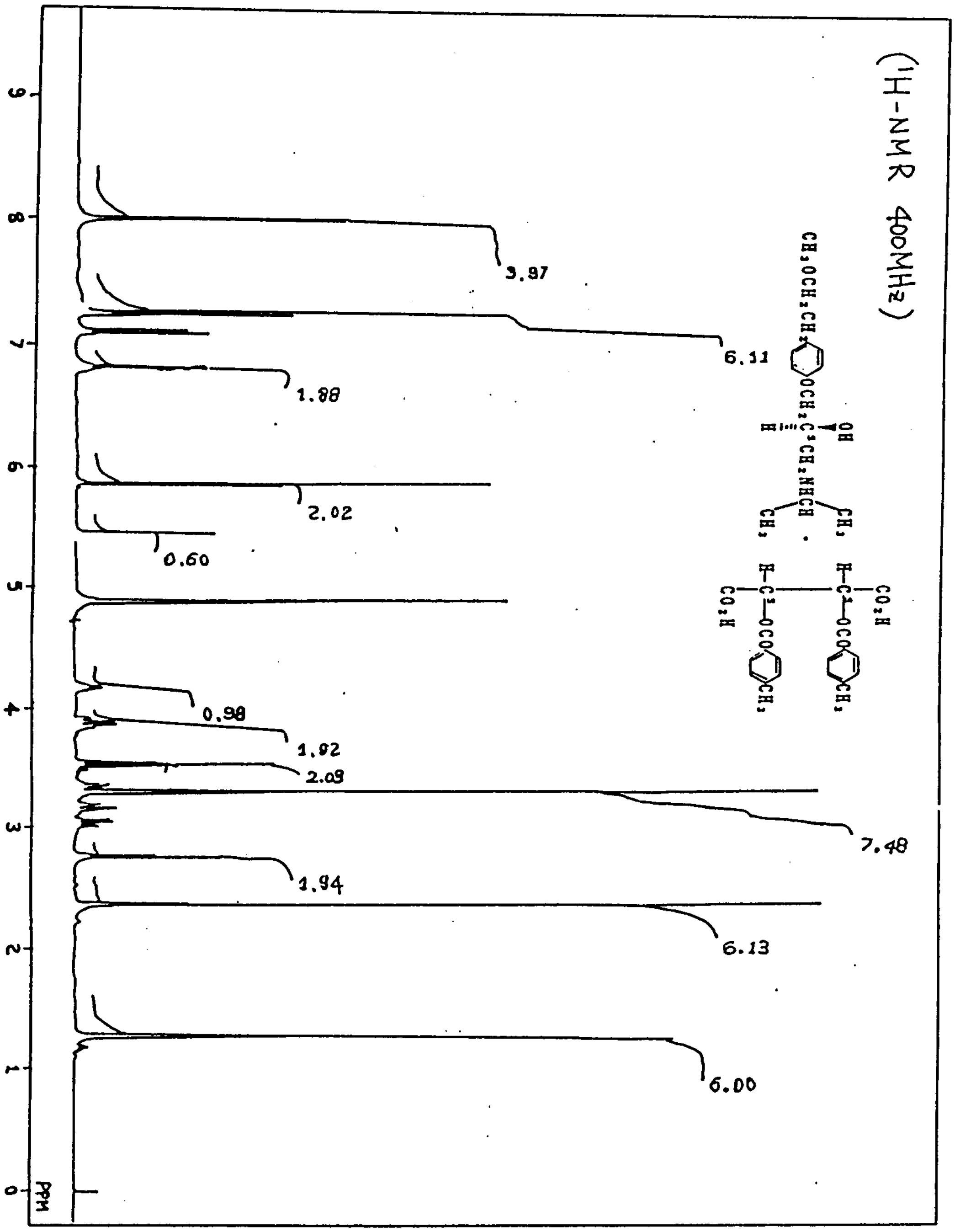
($^1$H-NMR 400MHz)
3.97
6.11
1.88
2.02
0.60
0.98
1.92
2.03
7.48
1.94
6.13
6.00
PPM
9
8
7
6
5
4
3
2
1
0

Fig. 2

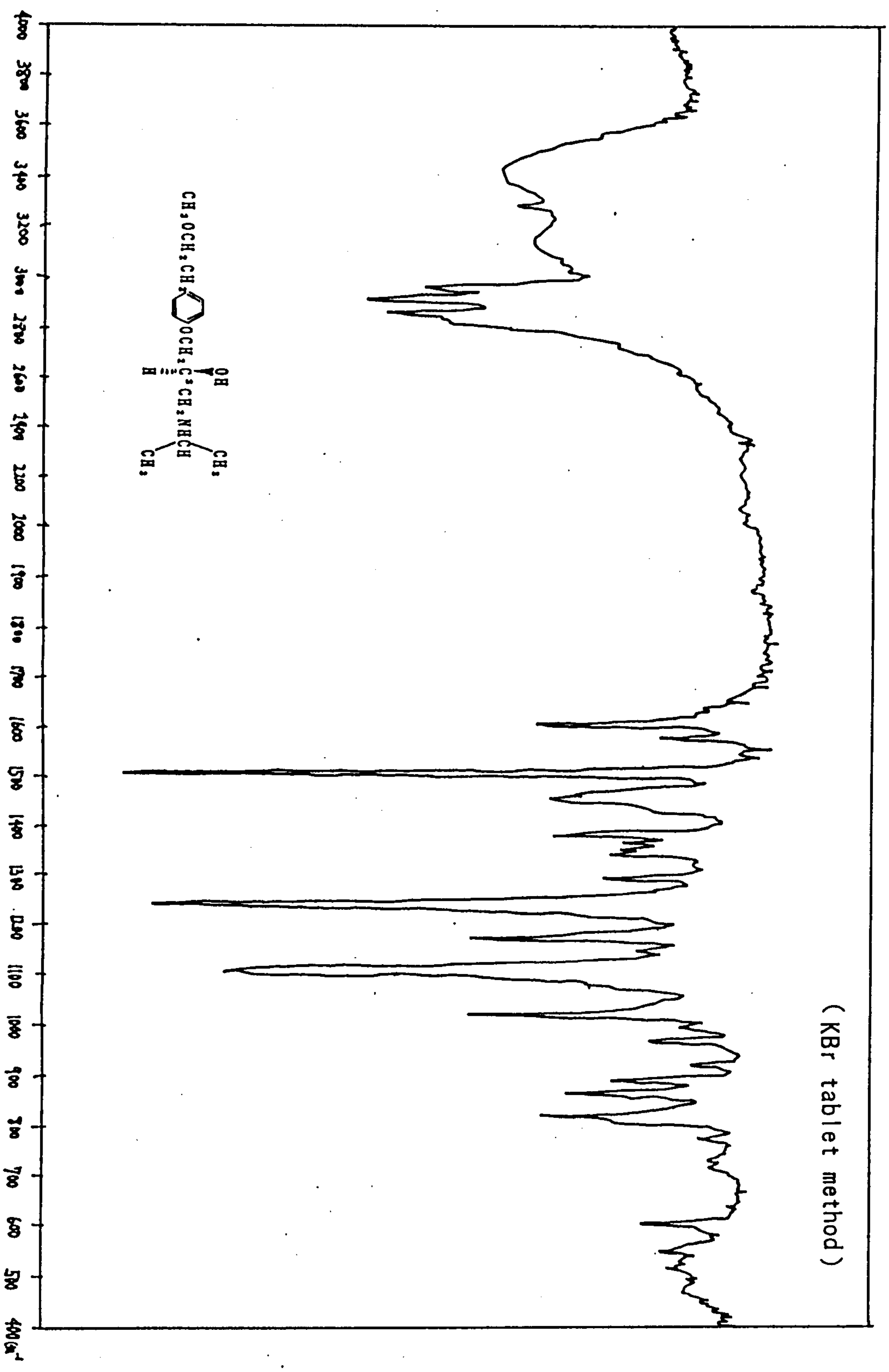


Fig. 3

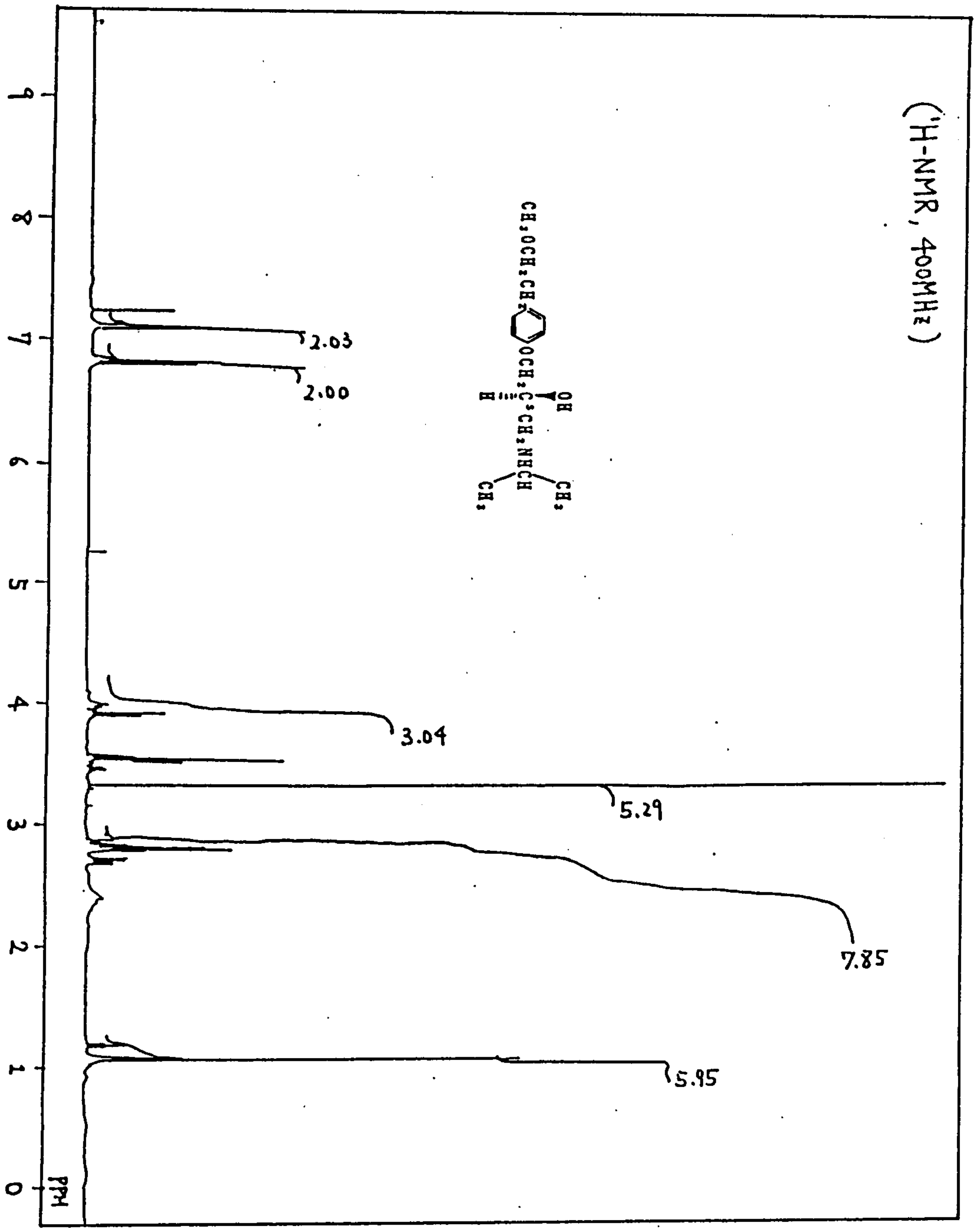


Fig. 4

European Patent Office

# EUROPEAN SEARCH REPORT

Application Number

EP 91 31 0439

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 050 885 (BLASINACHIM S.P.A.)<br>* claims; example 1 * | 1-6 | C07C213/10<br>C07C217/32<br>C07B57/00 |
| A | GB-A-1 136 919 (I.C.I.)<br>* whole document * | 1-6 | |
| A | US-A-3 868 460 (KOPPE ET.AL.)<br>* column 2, line 63 - column 3, line 2; claims; examples * | 1-6 | |
| A | EP-A-0 193 227 (GIST-BROCADES N.V.)<br>* claims; examples * | 1-6 | |
| A | EP-A-0 179 031 (AKTIEBOLAGET HÄSSLE)<br>* claims; examples 2,3 * | 1-6 | |
| A | GB-A-2 127 020 (BRISTOL-MYERS)<br>* claims; example 34 * | 1-6 | TECHNICAL FIELDS SEARCHED (Int. Cl.5 )<br>C07C |

The present search report has been drawn up for all claims

| Place of search | Date of completion of the search | Examiner |
|---|---|---|
| THE HAGUE | 11 FEBRUARY 1992 | HELPS I.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)